# EUROPÄISCHE PATENTSCHRIFT

(11) **EP 0 496 725 B1**
(45) Veröffentlichungstag und Bekanntmachung des Hinweises auf die Patenterteilung: **11.12.1996**
(21) Anmeldenummer: 92890018.2
(22) Anmeldetag: 23.01.1992
(51) Int. Cl.: C12N 9/64, C12N 11/08, A61K 38/49, C12P 21/08

(54) **Komplex enthaltend den Gerinnungsfaktor IX**
Complex containing coagulation factor IX
Complexe contenant le facteur de coagulation IX

(30) Priorität: 25.01.1991 AT 163/91
(43) Veröffentlichungstag der Anmeldung: 29.07.1992
(73) Patentinhaber: IMMUNO Aktiengesellschaft, A-1221 Wien (AT)
(72) Erfinder: Linnau, Yendra, Dr., A-1224 Wien (AT); Sazgary, Maria, Dipl.-Ing., A-1210 Wien (AT)
(74) Vertreter: Wolfram, Gustav, Dipl.-Ing.

(56) Entgegenhaltungen:
- EP-A- 0 229 026
- EP-A- 0 317 376
- DE-C- 3 914 869
- FOLIA HAEMATOLOGICA Bd. 107, Nr. 1, 1979, LEIPZIG Seiten 148 - 151 VUKOVICH, T. ET AL 'Separation of human blood clotting factors from fibrinogen and other plasma proteins by chromatography on butyl-sepharose'
- SCOPES P.K. 'Protein purification. Principles and practice' 1987 , SPRINGER-VERLAG , NEW YORK
- Pschyrembel-Klinisches Wörterbuch, 255 Auflage, Seite 215-216
- Römpp's Chemielexikon, 9 Auflage, Seite 4951 und 4105

## Beschreibung

Die Erfindung betrifft einen Komplex, welcher den Gerinnungsfaktor IX enthält, sowie ein Verfahren zur Herstellung eines Faktor IX-hältigen, pharmazeutischen Präparates.

Der Gerinnungsfaktor IX wird zur Substitutionsbehandlung von Patienten verwendet, welche an Hämophilie B leiden. Diese Blutungskrankheit resultiert aus einem Mangel an Faktor IX.

Zur Substitutionsbehandlung werden Faktor IX-Konzentrate verwendet, die meist noch zusätzlich die Gerinnungsfaktoren II, VII, IX und X (Prothrombinkomplex) enthalten. Die in derartigen Konzentraten enthaltenen Gerinnungsfaktoren zählen zu den Vitamin-K-abhängigen Faktoren und sind aufgrund ihres ähnlichen Aufbaus miteinander vergesellschaftet. Für Bluter, die an Hämophilie B leiden, bedeutet aber jede Zufuhr von anderen Gerinnungsfaktoren als Faktor IX eine Belastung. Deshalb ist man bestrebt, hochangereicherte Faktor IX-Konzentrate alternativ zu den Kombinationspräparaten in der Therapie einzusetzen.

Ausgangsmaterial zur Herstellung von Faktor IX-Präparaten ist menschliches Plasma, das den Faktor IX aber nur in geringen Mengen enthält (4 µg/ml). Zur Gewinnung des Faktors IX müssen daher nicht nur große Mengen Plasma verarbeitet werden, es müssen auch die störenden Begleitproteine so weit wie möglich abgetrennt werden. Erschwerend kommt hinzu, daß diese Begleitproteine ähnliche physikalisch-chemische Eigenschaften aufweisen.

Eine weitere Schwierigkeit besteht darin, daß das Endprodukt im Zuge der Aufarbeitung von infektiösen Agentien befreit werden muß, da das Ausgangsmaterial z.B. Hepatitis-Viren oder HIV enthalten kann. Dabei muß aber jeder einzelne Verfahrenschritt und jede Virusinaktivierung so durchgeführt werden, daß die biologische Aktivität des Faktors IX möglichst erhalten bleibt. Seit Jahren wird versucht, ein Verfahren zu finden, das die genannten Anforderungen optimal erfüllt.

Die Anreicherung des Faktors IX bei gleichzeitiger Abreicherung der anderen Faktoren durch chromatographische Verfahren gelingt naturgemäß umso besser, je spezifischer die Wechselwirkung des Gerinnungsfaktors IX mit der stationären Phase ist.

Für die Herstellung einer Faktor IX-hältigen Fraktion aus die Faktoren II, VII, IX und X enthaltenden wässerigen Mischungen sind bereits ionenaustausch- und affinitätschromatographische Verfahren bekannt. Gemäß dem in der DE-C - 39 14 869 beschriebenen Verfahren wird eine Faktor IX-hältige Fraktion gewonnen, indem der Faktor IX zunächst an einem eine α-Hydroxyl-Amino-Gruppe tragenden Adsorbens adsorbiert wird. Die Elution des adsorbierten Faktors IX erfolgt durch Erhöhung der Amin- oder der Salzkonzentration, was auf ionische Bindungskräfte zwischen dem Faktor IX und dem Adsorbens hindeutet. Im Anschluß an diese erste Anreicherungsstufe wird eine Reinigung mittels Chromatographie an einer sulfatiertes Kohlehydrat tragenden Matrix angeschlossen.

Affinitätschromatographische Verfahren zeichnen sich durch eine hohe Spezifität aus. Faktor IX wird an ein Trägermaterial via Antikörper oder anderen Gruppen mit hoher Affinität zu Faktor IX, z.B. Heparin, adsorbiert, gewaschen und eluiert. Beispielsweise wird nach dem Verfahren der EP-A - 317 376 Faktor IX an immobilisiertem Heparin adsorbiert. Die Elution des Faktors IX erfolgt durch Erhöhung der Salzkonzentration im Puffer. Ähnliches gilt für Antikörper gegen Faktor IX als Affinitätsträger.

In der EP-A-229 026 wird Faktor IX an einen immobilisierten Antikörper adsorbiert und durch einen Salzgradienten eluiert. Ein Problem besteht darin, daß bei den angewandten Elutionsbedingungen nicht nur der Faktor IX dissoziiert, sondern auch Heparin bzw. Antikörper, die in der Folge als Verunreinigungen im Endprodukt vorliegen.

Weiters ist bekannt, daß ein Gerinnungsfaktorenkonzentrat, enthaltend die Faktoren II, VII, IX und X, durch Chromatographie an einer hydrophoben Matrix von Virus-Antigenen befreit werden kann, indem die genannten Antigene an der Matrix hydrophob gebunden werden, während die Gerinnungsfaktoren unter Erhaltung ihrer biologischen Aktivität die Matrix passieren (J.Vir.Meth., 3, 213-228 (1981)).

In der Folia Haematol., Leipzig 107 (1979) I, S.148-151 wird die Trennung von menschlichen Blutgerinnungsfaktoren von Fibrinogen und Faktor VIII durch Butylsepharosechromatographie beschrieben.

Der Erfindung liegt die Aufgabe zugrunde, ein chromatographisches Verfahren bereitzustellen, das die genannten Nachteile nicht aufweist und eine leistungsfähige Anreicherung des Faktors IX aus einer wässerigen Mischung ermöglicht, welche noch weitere Proteine, insbesondere Plasmazymogene oder Vitamin K-abhängige Proteine, enthält.

Erfindungsgemäß wird ein Gerinnungsfaktor IX-Komplex zur Verfügung gestellt, welcher aus einem Träger auf Basis eines Polymeren mit hydrophoben Gruppen besteht, an den Faktor IX in - bezogen auf die Aktivität in Plasma - mindestens (2858 x 76%) : (3669 x 20%)-facher Aktivität gegenüber Plasmazymogenen bzw. Vitamin K-abhängigen Proteinen gebunden ist. Der Faktor IX kann im erfindungsgemäßen Komplex auch aus einer wässerigen, neben dem Faktor IX noch mindestens ein Vitamin K-abhängiges Protein, wie Protein C, Protein S oder die Faktoren II, VII und X enthaltenden Mischung, oder aus einer wässerigen Mischung, welche den Prothrombinkomplex enthält, selektiv gebunden vorliegen.

Die Erfindung beruht auf der Erkenntnis, daß Gerinnungsfaktor IX an einer hydrophobe Seitenketten aufweisenden Matrix aus einer wässerigen Lösung, welche eine hohe Leitfähigkeit aufweist, selektiv, d.h. in Gegenwart eines Plasmazymogens oder eines Vitamin K-abhängigen Proteins, adsorbiert. Das ist insofern überraschend, da die genannten Proteine ähnliche physikalisch-chemische Eigenschaften besitzen. Diese selektive Adsorbierbarkeit des Faktors IX kann zur Anreicherung des Faktors IX herangezogen werden.

Eine bevorzugte Ausführungsform des erfindungsgemäßen Komplexes ist dadurch gekennzeichnet, daß der Faktor IX in mindestens fünffacher Aktivität gegenüber einem anderen vorhandenen Vitamin K-abhängigen Protein enthalten ist.

Der erfindungsgemäße Komplex ist vorzugsweise einer Behandlung zur Inaktivierung etwa vorhandener infektiöser Agentien unterzogen.

Der erfindungsgemäße Komplex kann hergestellt werden, indem eine wässerige Mischung, welche neben dem Faktor IX noch mindestens ein Plasmazymogen enthält und eine Leitfähigkeit von mindestens 30 mS, vorzugsweise zwischen 60 und 120 mS, aufweist, mit einem Träger auf Basis eines Polymeren mit hydrophoben Gruppen in Kontakt gebracht wird, um den Faktor IX zu komplexieren, worauf der gebildete Komplex gegebenenfalls gewaschen und gegebenenfalls einer Inaktivierung von etwa vorhandenen infektösen Agentien unterzogen wird. Erfindungsgemäß wird der Faktor IX somit durch die Technik der hydrophoben Chromatographie angereichert. Vorteilhaft wird die Komplexbildung in Gegenwart eines Detergens durchgeführt.

Die Erfindung betrifft auch ein Verfahren zur Herstellung eines pharmazeutischen Präparates enthaltend den Gerinnungsfaktor IX, das dadurch gekennzeichnet ist, daß der Faktor IX aus dem erfindungsgemäßen Komplex eluiert wird, wonach das Faktor IX-hältige Eluat zu einer pharmazeutischen Präparation aufgearbeitet wird. Die Elution wird - wie für die hydrophobe Chromatographie üblich - bei niedrigeren Leitfähigkeiten durchgeführt als die Adsorption. Zweckmäßigerweise wird die Elution bei einer Leitfähigkeit durchgeführt, welche maximal 20 % niedriger liegt.

Eine bevorzugte Ausführungsform des erfindungsgemäßen Verfahrens besteht darin, daß
- Plasma oder eine Faktor IX-hältige Plasmafraktion einer Anionenaustauscherchromatographie unterworfen wird, wobei Faktor IX gebunden wird,
- der gebundene Faktor IX eluiert wird,
- der eluierte Faktor IX in Gegenwart eines Detergens einer Anionenaustauscherchromatographie unterworfen wird, wobei Faktor IX gebunden wird,
- der gebundene Faktor IX eluiert wird,
- das Faktor IX-hältige Eluat einer Inaktivierung etwa vorhandener infektiöser Agentien unterzogen wird, und
- mittels hydrophober Chromatographie Faktor IX abgetrennt und zu einem pharmazeutischen Präparat verarbeitet wird.

Der erfindungsgemäße Komplex kann weiters zur Gewinnung monoklonaler oder polyklonaler Antikörper verwendet werden, welche gegen den Faktor IX gerichtet sind.

Mit den nachfolgenden Beispielen wird die Erfindung noch näher erläutert.

### Beispiel 1:

Aus 10 Liter menschlichem Blutplasma wird der Prothrombinkomplex (die Blutgerinnungsfaktoren II, VII, IX und X) nach der Methode von Brummelhuis (Methods of Plasma Protein Fractionation, ed JM Curling, pp 117, Acad. Press. 1980) durch Adsorption an DEAE-Sephadex gewonnen. 50 ml Prothrombinkomplex (3669 I.E. Faktor II, 2858 I.E. Faktor IX und 2734 I.E. Faktor X) werden diafiltriert, um die Zusammensetzung des Puffers A (1000 mM NaCl, 15 mM Natriumcitrat, pH: 7) zu erreichen.

Eine Säule mit 30 ml eines hydrophilen Vinylpolymers mit Butyl-Gruppen (Fractogel TSK-Butyl (MERCK, Darmstadt)) wird mit 100 ml Puffer A equilibriert und der Prothrombinkomplex mit einer Flußrate von 360 ml/h aufgetragen. Nach dem Waschen des beladenen Gels mit 350 ml Puffer A wird die Faktor IX-hältige Fraktion mittels 180 ml Puffer B (400 mM NaCl) eluiert.

Die Ausbeute für den Faktor IX lag bei 76 % der Ausgangsaktivität. Die spezifische Aktivität des Faktors IX betrug 19,7 I.E. Faktor IX/mg Protein. Die Faktoren II und X konnten zu weniger als 20 % der Ausgangsaktivität nachgewiesen werden.

### Beispiel 2:

50 ml Prothrombinkomplex werden analog zum Beispiel 1 chromatographiert. Als Säulenmaterial wird jedoch 45 ml eines Methacrylat-Polymer mit Phenyl-Gruppen (Toyopearl Phenyl 650 M (TOSO HAAS, Stuttgart)) verwendet, welches mit 1,4 M Ammoniumsulfat equilibriert wurde. Die Elution des Faktors IX erfolgt mit 0,2 M Ammoniumsulfat.

Die Ausbeute für den Faktor IX lag bei 48 % der Ausgangsaktivität und die spezifische Aktivität betrug 27,2 I.E. Faktor IX/mg Protein.

### Beispiel 3:

150 ml Prothrombinkomplex werden mittels Dextransulfat vorgereinigt (Miletich et al., Analytical Biochemistry 105, 304 (1980)). 20 ml Eluat (912 I.E. Faktor IX, 160 I.E. Faktor X) werden analog zum Beispiel 1 auf eine Säule mit 20 ml eines Agarose-Polymer mit Octyl-Gruppen (Octyl-Sepharose-CL-4B (PHARMACIA, Schweden)) aufgetragen. Die Säule wurde zuvor mit 80 ml Puffer A equilibriert. Nach dem Waschen des beladenen Gels mit 120 ml Puffer A wird die Faktor IX-hältige Fraktion mit 80 ml einer 250 mmolaren NaCl-Lösung eluiert.

Die Ausbeute an Faktor IX lag bei 54 % der Ausgangsaktivität. Die spezifische Aktivität betrug 186 I.E. Faktor IX/mg Protein. Faktor X war nur mehr in Spuren vorhanden.

### Beispiel 4:

50 ml Prothrombinkomplex werden analog zu Beispiel 1 mittels hydrophober Chromatographie getrennt. Unmittelbar vor dem Auftragen auf 35 ml eines hydrophilen Vinylpolymer mit Butyl-Gruppen (Fractogel TSK-Butyl) wurde der Prothrombinkomplex noch zusätzlich mit 2 % eines Polyoxyäthylen-20-sorbitan-monooleates Tween 80 und 0,01 % Tri-(n-butyl)phosphat nach dem in der US-A - 4,820.805 beschriebenen Verfahren behandelt.

Die Ausbeute an Faktor IX lag bei 83 % der Ausganqsaktivität und die spezifische Aktivität betrug 24,1 I.E. Faktor IX/mg Protein.

### Beispiel 5:

Um eventuelle Krankheitserreger zu inaktivieren, werden 2 g des lyophilisierten Prothrombinkomplex nach dem in der EP-A - 0 159 311 beschriebenen Verfahren 10 Stunden lang bei 60°C unter Erhöhung des partiellen Wasserdampfdrucks erhitzt. Anschließend wird die hydrophobe Chromatographie an 20 ml eines Agarose-Polymer mit Octyl-Gruppen (Octyl-Sepharose-CL-4B) durchgeführt.

Die Ausbeute an Faktor IX lag bei 63 % der Ausgangsaktivität. Die spezifische Aktivität betrug 32 I.E. Faktor IX/mg Protein.

## Patentansprüche

1. Gerinnungsfaktor IX-Komplex bestehend aus einem Träger auf Basis eines Polymeren mit hydrophoben Gruppen, an den Faktor IX in - bezogen auf die Aktivität in Plasma - mindestens (2858 x 76%) : (3669 x 20%)-facher Aktivität gegenüber anderen Plasmazymogenen bzw. Vitamin K-abhängigen Proteinen gebunden ist.

2. Komplex nach Anspruch 1, dadurch gekennzeichnet, daß das Vitamin K-abhängige Protein aus der Gruppe Protein C, Protein S, sowie Faktor II, VII und X, ausgewählt ist.

3. Komplex nach Anspruch 2, dadurch gekennzeichnet, daß der Faktor IX aus einer wässerigen Mischung gebunden ist, welche den Prothrombinkomplex enthält.

4. Komplex nach einem der Ansprüche 2 oder 3, in welchem der Faktor IX in mindestens fünffacher Aktivität gegenüber einem anderen Vitamin K-abhängigen Protein enthalten ist.

5. Komplex nach einem der Ansprüche 1 bis 4, behandelt zur Inaktivierung etwa vorhandener infektiöser Agentien.

6. Verfahren zur Herstellung eines Komplexes bestehend aus einem Träger auf Basis eines Polymeren mit hydrophoben Gruppen, an den Faktor IX gebunden ist, dadurch gekennzeichet, daß eine wässerige Mischung, welche neben dem Faktor IX noch mindestens ein Plasmazymogen enthält und eine Leitfähigkeit von mindestens 30 mS, vorzugsweise zwischen 60 und 120 mS, aufweist, mit einem Träger auf Basis eines Polymeren mit hydrophoben Gruppen in Kontakt gebracht wird, um den Faktor IX zu komplexieren, worauf der gebildete Komplex gegebenenfalls gewaschen und gegebenenfalls einer Inaktivierung von etwa vorhandenen infektiösen Agentien unterzogen wird.

7. Verfahren nach Anspruch 6, dadurch gekennzeichnet, daß die Komplexbildung in Gegenwart eines Detergens durchgeführt wird.

8. Verfahren zur Herstellung eines pharmazeutischen Präparates enthaltend den Gerinnungsfaktor IX, dadurch gekennzeichnet, daß der Faktor IX aus einem Komplex nach einem der Ansprüche 1 bis 5 oder hergestellt nach Anspruch 6 eluiert wird, wonach das Faktor IX-hältige Eluat zu einer pharmazeutischen Präparation aufgearbeitet wird.

9. Verfahren nach Anspruch 8, dadurch gekennzeichnet, daß die Elution bei einer Leitfähigkeit durchgeführt wird, welche maximal 20 % unter derjenigen liegt, welche zur Komplexierung des Faktors IX gemäß Anspruch 6 angewendet wurde.

10. Verfahren zur Herstellung eines pharmazeutischen Präparates, enthaltend den Gerinnungsfaktor IX, gekennzeichnet durch die Kombination der Maßnahmen, daß
- Plasma oder eine Faktor IX-hältige Plasmafraktion einer Anionenaustauscherchromatographie unterworfen wird, wobei Faktor IX gebunden wird,
- der gebundene Faktor IX eluiert wird,
- der eluierte Faktor IX in Gegenwart eines Detergens einer Anionenaustauscherchromatographie unterworfen wird, wobei Faktor IX gebunden wird,
- der gebundene Faktor IX eluiert wird,
- das Faktor IX-hältige Eluat einer Inaktivierung etwa vorhandener infektiöser Agentien unterzogen wird,
- das Faktor IX-hältige Eluat auf eine Leitfähigkeit von mindestens 30 mS, vorzugsweise zwischen 60 und 120 mS, eingestellt und mit einem Träger auf Basis eines Polymeren mit hydrophoben Gruppen in Kontakt gebracht wird, worauf sich ein Komplex bestehend aus dem Träger und Faktor IX bildet,
- der Komplex gegebenenfalls gewaschen und gegebenenfalls einer weiteren Inaktivierung von etwa vorhandenen infektiösen Agentien unterzogen wird, und
- Faktor IX aus dem Komplex eluiert und zu einem pharmazeutischen Präparat aufgearbeitet wird.

11. Verwendung eines Komplexes nach einem der Ansprüche 1 bis 5 zur Gewinnung monoklonaler oder polyklonaler Antikörper, welche gegen den Faktor IX gerichtet sind.

## Claims

1. A coagulation factor IX complex comprised of a carrier based on a polymer with hydrophobic groups, to which factor IX, based on the activity in plasma, is bound in an at least (2858 x 76%) : (3669 x 20%)-fold activity relative to other plasma zymogens or vitamin K-dependent proteins, respectively.

2. A complex according to claim 1, characterised in that the vitamin K-dependent protein is selected from the group of protein C, protein S, as well as factors II, VII and X.

3. A complex according to claim 2, characterised in that the factor IX is bound from an aqueous mixture containing the prothrombin complex.

4. A complex according to any one of claims 2 or 3, in which factor IX is contained in at least five-fold activity relative to another vitamin K-dependent protein.

5. A complex according to any one of claims 1 to 4, treated for inactivation of possibly present infectious agents.

6. A method for producing a complex comprised of a carrier based on a polymer with hydrophobic groups, to which factor IX is bound, characterised in that an aqueous mixture which contains at least one plasma zymogen in addition to factor IX and has a conductivity of at least 30 mS, preferably of between 60 and 120 mS, is contacted with a carrier based on a polymer with hydrophobic groups so as to complex factor IX, whereupon the complex formed optionally is washed and optionally is subjected to an inactivation of possibly present infectious agents.

7. A method according to claim 6, characterised in that the complex formation is carried out in the presence of a detergent.

8. A method for producing a pharmaceutical preparation containing coagulation factor IX, characterised in that the factor IX is eluted from a complex according to any one of claims 1 to 5 or produced according to claim 6, whereupon the factor IX-containing eluate is processed to a pharmaceutical preparation.

9. A method according to claim 8, characterised in that the elution is carried out at a conductivity which, at the most, is 20% below the one that had been used for complexing factor IX according to claim 6.

10. A method for producing a pharmaceutical preparation containing coagulation factor IX, characterised by the combination of the measures that
- plasma or a factor IX-containing plasma fraction is subjected to an anion exchange chromatography, thus binding factor IX,
- the bound factor IX is eluted,
- the eluted factor IX is subjected to an anion exchange chromatography in the presence of a detergent, thus binding factor IX,
- the bound factor IX is eluted,
- the factor IX-containing eluate is subjected to an inactivation of possibly present infectious agents,
- the factor IX-containing eluate is adjusted to a conductivity of at least 30 mS, preferably between 60 and 120 mS, and is contacted with a carrier based on a polymer with hydrophobic groups, whereupon a complex consisting of carrier and factor IX forms,
- the complex optionally is washed and optionally is subjected to a further inactivation of possibly present infectious agents, and
- factor IX is eluted from the complex and is processed to a pharmaceutical preparation.

11. The use of a complex according to any one of claims 1 to 5 for recovering monoclonal or polyclonal antibodies directed against factor IX.

## Revendications

1. Complexe du facteur IX de la coagulation consistant en un support à base d'un polymère à groupes hydrophobes auquel est lié le facteur IX en une activité, rapportée à l'activité dans le plasma, au moins (2858 x 76%) : (3 669 x 20 %) fois supérieure à d'autres zymogènes plasmatiques ou protéines dépendantes de la vitamine K.

2. Complexe selon la revendication 1, caractérisé en ce que la protéine dépendante de la vitamine K est choisie dans le groupe de la protéine C, de la protéine S et des facteurs II, VII et X.

3. Complexe selon la revendication 2, caractérisé en ce que le facteur IX est lié à partir d'un mélange aqueux qui contient le complexe de la prothrombine.

4. Complexe selon l'une des revendications 2 ou 3, dans lequel le facteur IX est contenu en une activité au moins cinq fois supérieure à une autre protéine dépendante de la vitamine K.

5. Complexe selon l'une des revendications 1 à 4 traité pour l'inactivation d'agents infectieux éventuellement présents.

6. Procédé de préparation d'un complexe consistant en un support à base d'un polymère à groupes hydrophobes auquel est lié le facteur IX, caractérisé en ce qu'un mélange aqueux qui, outre le facteur IX, contient également au moins un zymogène plasmatique et qui présente une conductivité d'au moins 30 mS, de préférence comprise entre 60 et 120 mS, est mis en contact avec un support à base d'un polymère à groupes hydrophobes pour complexer le facteur IX, après quoi le complexe formé est éventuellement lavé et éventuellement soumis à une inactivation d'agents infectieux éventuellement présents.

7. Procédé selon la revendication 6, caractérisé en ce que la complexation est réalisée en présence d'un détergent.

8. Procédé d'obtention d'une préparation pharmaceutique contenant le facteur IX de la coagulation, caractérisé en ce que le facteur IX est élué d'un complexe selon l'une des revendications 1 à 5 ou préparé selon la revendication 6, après quoi l'éluat contenant le facteur IX est mis sous forme d'une préparation pharmaceutique.

9. Procédé selon la revendication 8, caractérisé en ce que l'élution est réalisée à une conductivité qui est située au maximum à 20 % en deçà de celle qui a été utilisée pour la complexation du facteur IX selon la revendication 6.

10. Procédé d'obtention d'une préparation pharmaceutique contenant le facteur IX de la coagulation, caractérisé par la combinaison des mesures selon lesquelles :
- du plasma ou une fraction plasmatique contenant le facteur IX est soumis à une chromatographie sur échangeur d'anions de sorte que le facteur IX est lié,
- le facteur IX lié est élué,
- le facteur IX élué est soumis à une chromatographie sur échangeur d'anions en présence d'un détergent de sorte que le facteur IX est lié,
- le facteur IX lié est élué,
- l'éluat contenant le facteur IX est soumis à une inactivation d'agents infectieux éventuellement présents,
- l'éluat contenant le facteur IX est réglé à une conductivité d'au moins 30 mS, de préférence comprise entre 60 et 120 mS, et est mis en contact avec un support à base d'un polymère à groupes hydrophobes, après quoi il se forme un complexe consistant en le support et le facteur IX,
- le complexe est éventuellement lavé et éventuellement soumis à une inactivation supplémentaire d'agents infectieux éventuellement présents, et
- le facteur IX est élué du complexe et mis sous forme d'une préparation pharmaceutique.

11. Utilisation d'un complexe selon l'une des revendications 1 à 5 pour l'obtention d'anticorps monoclonaux ou polyclonaux qui sont dirigés contre le facteur IX.
